(19) Europäisches Patentamt
European Patent Office
Office européen des brevets

(11) Publication number: **0 136 479 B1**

(12) **EUROPEAN PATENT SPECIFICATION**

(45) Date of publication of patent specification: **17.10.90**

(21) Application number: **84109358.6**

(22) Date of filing: **07.08.84**

(51) Int. Cl.$^5$: **A 61 K 31/01,** A 61 K 35/64 // (A61K31/01, 35:64),(A61K35/64, 31:01)

(54) **Topical treatment of skin inflammatory disorders.**

(30) Priority: **10.08.83 US 521950**

(43) Date of publication of application:
**10.04.85 Bulletin 85/15**

(45) Publication of the grant of the patent:
**17.10.90 Bulletin 90/42**

(84) Designated Contracting States:
**AT BE CH DE FR GB IT LI LU NL SE**

(56) References cited:
**EP-A-0 123 637**
**FR-A-2 480 120**
**US-A-2 361 477**
**US-A-4 331 653**

**Merck Index, 1983, p. 144, 1020.**

(73) Proprietor: **Richardson-Vicks, Inc.**
**Ten Westport Road**
**Wilton, CT 06897 (US)**

(72) Inventor: **Gans, Eugene**
**5 Fairview Drive**
**Westport Connecticut 06880 (US)**
Inventor: **Nacht, Sergio**
**48 Wildwood Lane**
**Weston Connecticut 06883 (US)**
Inventor: **Yeung, David**
**506 Long Ridge Road**
**Stamford Connecticut 06810 (US)**

(74) Representative: **Vossius & Partner**
**Siebertstrasse 4 P.O. Box 86 07 67**
**D-8000 München 86 (DE)**

**EP 0 136 479 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

This invention relates to the discovery that a certain beeswax fraction affords an effective topical treatment for certain inflammatory skin conditions.

Beeswax is the yellow or white (bleached) wax obtained from the honeycomb of the bee and consists largely of myricyl palmitate, cerotic acid and esters, and some high-carbon paraffins and mono-unsaturated long-chain hydrocarbons (monoenes). Trace amounts of triacontanol, i.e., myricyl alcohol, may also be present although this alcohol is predominantly in the form of the palmitate ester.

It has now been found that a certain fraction of beeswax possesses substantial vasoconstrictive activity suitable for topically treating inflammation in humans or animals. This fraction consists essentially of a mixture of the non-polar saturated and mono-unsaturated long-chain hydrocarbons found in beeswax. Analysis of this fraction indicates the presence of about 90% w/w of saturated straight chain hydrocarbons with chain lengths ranging from $C_{21}$ to $C_{33}$, as represented by the formula:

$$CH_3—(CH_2)_n—CH_3 \qquad (I)$$

wherein n is an integer from 19 to 31, with the remainder of the fraction, about 10% w/w, being mono-unsaturated straight chain hydrocarbons of similar chain lengths as in Formula (I). The exact location of the single unsaturated bond in the latter monoenes has not been determined.

The aforementioned hydrocarbon fraction is substantially devoid, that is, containing less than 5% w/w of the polar constituents found in beeswax, such as, for example, myricyl palmitate, cerotic acid and its esters and the like.

According to D. T. Downing et al. Australian J. Chem. *14*: 253—263, 1961, the makeup of the naturally occurring hydrocarbons in beeswax is approximately as follows in percent by weight:

| n-Paraffin hydrocarbon carbon No. | Naturally occurring hydrocarbons | | |
|---|---|---|---|
| | Wax A | Wax B | Average |
| 19 | | | |
| 20 | 0.5 | 0.3 | 0.4 |
| 21 | 0.8 | 0.8 | 0.8 |
| 22 | 0.3 | 0.2 | 0.25 |
| 23 | 3.7 | 3.7 | 3.7 |
| 24 | 0.6 | 0.4 | 0.5 |
| 25 | 7.5 | 8.8 | 8.15 |
| 26 | 1.2 | 1.1 | 1.1 |
| 27 | 26.8 | 30.1 | 28.45 |
| 28 | 2.2 | 1.3 | 1.75 |
| 29 | 19.3 | 16.5 | 17.9 |
| 30 | 1.6 | 0.9 | 1.25 |
| 31 | 20.8 | 19.0 | 19.9 |
| 32 | 0.9 | 1.5 | 1.2 |
| 33 | 13.8 | 15.5 | 14.65 |
| | 100.0 | 100.0 | 100.00 |

The subject hydrocarbon fraction has been found to be therapeutically effective in treating inflammation of the skin, including acne. For purposes of this disclosure, the term "treating acne" is used to mean the temporary alleviation of the inflammation of the effected skin and other inflammatory signs and symptoms associated with acne.

In addition to treating acne, the superior vasoconstrictor activity of the subject hydrocarbon fraction affords its usage as an effective anti-inflammatory treatment for the following skin disorders: atopic dermatitis, atopic eczema, herpes simplex, shingles, poison ivy, poison oak, poison sumac and other skin allergic reactions, psoriasis and dandruff. As with acne, the term "anti-inflammatory treatment" or its equivalent is used to mean the temporary alleviation of the inflammation of the affected skin and other inflmmatory signs and symptoms associated with the particular skin disorder.

Suitable pharmaceutical carriers for the topical administration of the subject hydrocarbon fraction are non-polar pharmaceutical vehicles in conventional forms such as solutions, lotions, emulsions, ointments and gels, in which the pharmaceutical carrier merely provides a physical form for the effective topical application of the active hydrocarbon fraction to the skin. The therapeutic composition is prepared by simply mixing the desired therapeutically effective amount of the hydrocarbon fraction with the particular carrier according to conventional pharmaceutical compounding techniques.

By a "therapeutically effective amount" is meant an amount which is effective to alleviate the

inflammation of the dermatological condition and yet cause substantially no undesirable side effects (at a reasonable benefit/risk ratio). In general, the hydrocarbon fraction is therapeutically effective in from 0.1 to 10 percent by weight, based on the composition weight, with amounts from 0.5 to 5 percent by weight being preferred.

The isolation of the subject hydrocarbon fraction from beeswax is illustrated in the following example using column chromatography on silica gel with hexane as the elution solvent.

Example 1

A glass column 50 cm in length and 3 cm in diameter was dry-packed with Silica Gel 60 of particle size 0.063—0.200 mm (70—230 mesh ASTM); purchased from E. Merck Chemical Co. The Silica Gel was oven-dried at 120°C for 4 to 6 hours prior to use. 50 Grams of Beeswax (yellow wax obtained from honeycombs) was dissolved in 500 mls of warm hexane and the mixture was passed through the column at ambient temperature. The eluent was collected in a sutiable glass vessel. The column was then eluted with an additional 1000 mls of hexane. The eluents were pooled and evaporated to dryness under vacuum using a rotoevaporator, affording the hydrocarbon fraction of this invention as off-white crystals, m.p.=51°C.

In addition to hexane, which is preferred, other non-polar organic solvents may be used to extract the hydrocarbon fraction from beeswax such as, for example, an aliphatic alkane such as pentane, and heptane an aromatic hydrocarbon such as benzene, toluene, and xylene; an ether such as diethyl ether, and dioxane; and tetrahydrofuran. With such aprotic solvents as eluents, it is preferred to use a polar stationing phase such as, for example, silica gel, in the chromatographic separation step.

Accordingly, the subject hydrocarbon fraction may be derived from beeswax by:

a) dissolving beeswax in an organic aprotic solvent;

b) chromatographically separating out of said beeswax solution substantially all of the polar constituents of beeswax;

c) collecting the chromatographic eluent containing the non-polar hydrocarbon fraction; and

d) evaporating the organic solvent from said eluent to yield the substantially non-polar hydrocarbon fraction.

The chemical analysis of the hydrocarbon fraction obtained from Example 1 is demonstrated in the following two examples.

Example 2

Thin layer chromatography (TLC)

5 microliters of chloroform containing 10 to 20 µg of the material to be tested is spotted on a 20×20 cm, 250 micron silica gel G plate. The plate is developed in toluene once, air dried and sprayed with 50% sulfuric acid. The plate is then charred on a hot plate at 220°C. All carbon containing materials appear as dark brown to black spots and the amount of carbon containing materials correlates with the intensity of the spot. Identification of compounds is accomplished by comparing the mobility of compounds to that of authentic standards. Analysis of the hydrocarbon fraction obtained from Example 1 by this TLC technique revealed the presence of >95% hydrocarbons with <5% polar materials at the origin.

Example 3

Gas liquid chromatography (GLC)

GLC analysis was performed on a column packed with 3% SE-30 (80/100 mesh). Temperature programming was from 120°C—300°C at a rate of 5°C/minute. 2 Microliters of the sample was injected and detection was accomplished by flame ionization. Identification of the chain length distribution of the beeswax derived hydrocarbons was accomplished by direct comparison of its chromatogram with that of a series of normal paraffins of even carbon number $C_{14}$ through $C_{34}$ and the plotting or retention times against the carbon number. Analysis of the hydrocarbon fraction obtained from Example 1 by this GLC technique shows a content of about 90% saturated straight chain hydrocarbons with chain length ranging from $C_{21}$ to $C_{33}$ and about 10% mono-unsaturated hydrocarbons of similar chain length.

The instant invention thus provides a pharmaceutical composition for alleviating inflammation associated with skin disorders comprising a therapeutically effective amount of a substantially non-polar hydrocarbon fraction derived from beeswax and a pharmaceutical carrier suitable for topical administration, said beeswax fraction consisting essentially of (i) more than 95 percent by weight of a hydrocarbon mixture consisting of about 90 percent by weight of saturated straight chain hydrocarbons and about 10 percent by weight of mono-unsaturated hydrocarbons, wherein the chain length of said hydrocarbons is from $C_{21}$ to $C_{33}$, and (ii) less than 5 percent by weight of polar constituents in beeswax.

A particularly suitable pharmaceutical carrier in ointment form for purposes of this invention is Hydrophilic Ointment U.S.P., an oil-in-water emulsion ointment base having the formulation:

| | |
|---|---|
| Methylparaben | 0.25 g |
| Propylparaben | 0.15 g |
| Sodium lauryl sulfate | 10 g |
| Propylene glycol | 120 g |
| Stearyl alcohol | 250 g |
| White petrolatum | 250 g |
| Purified water | 370 g |
| To make about | 1000 g |

The stearyl alcohol and the white petrolatum are melted on a steam bath and warmed to about 75°C. The other ingredients are dissolved in the purified water and also heated to 75°C. The petrolatum phase is then added to the water phase with mixing until the mixture congeals. The resultant ointment is cooled to room temperature.

The vasoconstriction activity of the subject hydrocarbon fraction is demonstrated in the following in-vivo vasoconstrictor assay, which is a modification of the Stoughton-McKenzie Vasoconstrictor Assay described in "Mthod for Comparing Percutaneous Absorption of Steroids", Arch. Derm. 86: 608—610, 1962.

The test was performed on a defined area of the volar aspect of the forearm in 5 subjects. The test formulations were applied under semi-occlusion to maximize differences in activity. Thus, test formulations were saturated on the absorbent cushion pad of 3/4 inch bandages (Curity Curad Sheer Bandages) and the bandages were taped to the forearm with no more than 5 bandages per forearm. The bandages were left on the forearm for 24 hours and then removed. The treatment sites were washed with soap and water to remove any excess material still on the skin surface. After 1 hour, the resulting blanching or whitening of the skin was then scored by two judges using the following scoring system:

0=No blanching
1=Barely perceptible blanching
2=Distinct blanching with well defined outline
3=Strong blanching

An increase in blanching reflects a corresponding increase in vasoconstriction activity.

Example 4

The experimental results on the activity of the subject hydrocarbon fraction in the foregoing vasoconstruction assay are set forth below. For comparative purposes, a potent commercially available (Syntex Laboratories, Inc.) steroid anti-inflammatory product, LIDEX® Cream, containing 0.05% of the active anti-inflammatory compound fluocinonide, was used as a positive control. Also included in the test were an alcohol extract from beeswax containing the polar constituents of beeswax itself, and triacontanol (myricyl alcohol) Reference Standard (>99% pure). The results tabulated below are averages of at least five subjects. Products B through E were tested at 1% w/w concentration in the previously described Hydrophilic Ointment U.S.P.

| Product | Blanching |
|---|---|
| A. LIDEX® Cream 0.05% | 2.5 |
| B. Hydrocarbon fraction obtained from Example 1 | 2.3 |
| C. Alcohol extract of beeswax | 1.1 |
| D. Triacontanol Ref. Std. | 0.5 |
| E. Beeswax (yellow) | 1.0 |
| F. Hydrophilic Ointment U.S.P. | 0.6 |

As the results indicate, LIDEX® Cream 0.05% induced the highest vasoconstrictive effect with close to a maximum score of 2.5. The subject hydrocarbon fraction from beeswax scored 2.3 in the blanching scale, which, although slightly lower than that of LIDEX® Cream 0.05%, is significantly higher than any of the other materials tested. The alcohol extract obtained from beeswax and the unfractionated beeswax are equally effective with a blanching score of 1.1 and 1.0 respectively, indicating only very mild blanching was observed. The hydrophilic ointment vehicle induced a slight blanching effect with a score of 0.6. Pure triacontanol (Reference Standard) was found to have the lowest activity with a score of 0.5; suggesting that it has little or no vasoconstrictor activity.

In view of its marked vasoconstrictor activity, the subject hydrocarbon fraction is deemed to be of value as a therapeutic agent for treatment of inflammatory skin disorders. When the compositions of the present

invention are used in the treatment of such disorders, the amount of composition typically applied and treatment regimen will vary, depending upon, for example, the particular disorder being treated and its severity, the frequency of application and the area of the body which is afflicted.

For example, when the compositions of this invention are used in the topical treatment of acne, the preferred treatment will comprise applying a therapeutically effective amount of the composition to the afflicted situs on the skin. Generally, a therapeutically effective amount would be from 1 mg/cm$^2$ to 10 mg/cm$^2$ of the composition per day. It is preferred to cleanse the skin prior to treatment. The treatment is more effective if topical applications are made 2 to 4 times daily.

**Claims for the Contracting States: BE, CH, DE, FR, GB, IT, LI, LU, NL, SE**

1. A pharmaceutical composition for alleviating inflammation associated with skin disorders, characterized in that it comprises a hydrocarbon mixture consisting of about 90 percent by weight of saturated straight chain hydrocarbons and about 10 percent by weight of mono-unsaturated hydrocarbons found in beeswax, wherein the chain length of said hydrocarbons is from $C_{21}$ to $C_{33}$, in a pharmaceutical carrier suitable for topical administration.

2. A pharmaceutical composition according to claim 1, characterized in that the hydrocarbon mixture is derived from beeswax and the composition contains in addition less than 5 percent by weight based on oha total amount of beeswax components of polar constituents of beeswax.

3. A pharmaceutical composition according to claim 2, characterized in that it comprises from 0.1 to 10 percent by weight, based on the composition weight, of the hydrocarbon fraction derived from beeswax.

4. A pharmaceutical composition according to claim 2 or 3 for alleviating inflammation associated with acne.

5. Use of a hydrocarbon mixture consisting of about 90 percent by weight of saturated straight chain hydrocarbons and about 10 percent by weight of mono-unsaturated hydrocarbons wherein the chain length of said hydrocarbons is from $C_{21}$ to $C_{33}$, for the production of a pharmaceutical composition suitable for topical administration for topical use for alleviating inflammation associated with skin disorders, especially acne.

**Claims for the Contracting State: AT**

1. Method for the preparation of a pharmaceutical composition for alleviating inflammation associated with skin disorders, characterized in that a hydrocarbon mixture consisting of about 90 percent by weight of saturated straight chain hydrocarbons and about 10 percent by weight of mono-unsaturated hydrocarbons found in beeswax, wherein the chain length of said hydrocarbons is from $C_{21}$ to $C_{33}$, is combined with a pharmaceutical carrier suitable for topical administration.

2. The method according to claim 1, characterized in that the hydrocarbon mixture is derived from beeswax and in addition less than 5 percent by weight based on the total amount of beeswax components of polar constituents of beeswax are added to the composition.

3. The method according to claim 2, characterized in that from 0.1 to 10 percent by weight, based on the combination weight, of the hydrocarbon fraction derived from beeswax are added to the composition.

4. Use of a hydrocarbon mixture consisting of about 90 percent by weight of saturated straight chain hydrocarbons and about 10 percent by weight of mono-unsaturated hydrocarbons wherein the chain length of said hydrocarbons is from $C_{21}$ to $C_{33}$, for the production of a pharmaceutical composition suitable for topical administration for topical use for alleviating inflammation associated with skin disorders, especially acne.

**Patentansprüche für die Vertragsstaaten: BE, CH, DE, FR, GB, IT, LI, LU, NL, SE**

1. Arzneimittel zur Linderung von mit Hauterkrankungen verbundener Entzündung, dadurch gekennzeichnet, daß es ein Kohlenwasserstoff-Gemisch, das aus etwa 90 Gew.-% gesättigter geradkettiger Kohlenwasserstoffe und etwa 10 Gew.-% einfach ungesättigter Kohlenwasserstoffe besteht, die in Bienenwachs gefunden werden, wobei die Kettenlänge der Kohlenwasserstoffe von $C_{21}$ bis $C_{33}$ reicht, in einem zur lokalen Verabreichung geeigneten pharmazeutischen Träger umfaßt.

2. Arzneimittel nach Anspruch 1, dadurch gekennzeichnet, daß das Kohlenwasserstoff-Gemisch von Bienenwachs abgeleitet ist und das Mittel zusätzlich weniger als 5 Gew.-%, bezogen auf die Gesamtmenge der Bienenwachs-Komponenten, polare Bestandteile von Bienenwachs enthält.

3. Arzneimittel nach Anspruch 2, dadurch gekennzeichnet, daß es 0,1 bis 10 Gew.-%, bezogen auf die Masse des Mittels, der von Bienenwachs abgeleiteten Kohlenwasserstoff-Fraktion enthält.

4. Arzneimittel nach Anspruch 2 oder 3 zur Linderung von mit Akne verbundener Entzündung.

5. Verwendung eines Kohlenwasserstoff-Gemisches, das aus etwa 90 Gew.-% gesättiger geradkettiger Kohlenwasserstoffe und etwa 10 Gew.-% ein fach ungesättiger Kohlenwasserstoffe besteht, wobei die Kettenlänge der Kohlenwasserstoffe von $C_{21}$ bis $C_{33}$ reicht, zur Herstellung eines Arzneimittels, das zur lokalen Verabreichung für lokale Anwendung zur Linderung von mit Hauterkrankungen, insbesondere Akne, verbundener Entzündung geeignet ist.

**Patentansprüche für den Vertragsstaat: AT**

1. Verfahren zur Herstellung eines Arznemittels zur Linderung von mit Hauterkrankungen verbundener Entzündung, dadurch gekennzeichnet, daß ein Kohlenwasserstoff-Gemisch, das aus etwa 90 Gew.-% gesättiger geradkettiger Kohlenwasserstoffe und etwa 10 Gew.-% einfach ungesättiger Kohlenwasserstoffe besteht, die in Bienenwachs gefunden werden, wobei die Kettenlänger der Kohlenwasserstoffe von $C_{21}$ bis $C_{33}$ reicht, mit in einem zur lokalen Verabreichung geeigneten pharmazeutischen Träger verbunden wird.

2. Verfahren nach Anspruch 1, dadurch gekennzeichnet, daß das Kohlenwasserstoff-Gemisch von Bienenwachs abgeleitet ist und daß zusätzlich weniger als 5 Gew.-%, bezogen auf die Gesamtmenge der Bienenwachs-Komponenten, polare Bestandteile von Bienenwachs dem Mittel zugegeben werden.

3. Verfahren nach Anspruch 2, dadurch gekennzeichnet, daß 0,1 bis 10 Gew.-%, bezogen auf die Masse des Mittels, der von Bienenwachs abgeleiteten Kohlenwasserstoff-Fraktion dem Mittel zugegeben werden.

4. Verwendung eines Kohlenwasserstoff-Gemisches, das aus etwa 90 Gew.-% gesättiger geradkettiger Kohlenwasserstoffe und etwa 10 Gew.-% einfach ungesättiger Kohlenwasserstoffe besteht, wobei die Kettenlänge der Kohlenwasserstoffe von $C_{21}$ bis $C_{33}$ reicht, zur Herstellung eines Arzneimittels, das zur lokalen Verabreichung für lokale Anwendung zur Linderung von mit Hauterkrankungen, insbesondere Akne, verbundener Entzündung geeignet ist.

**Revendications pour les Etats Contractants: BE, CH, DE, FR, GB, IT, LI, LU, NL, SE**

1. Composition pharmaceutique vour soulager l'inflammation associée aux troubles de la peau, caractérisé en ce que comprend un mélange d'hydrocarbures comprenant d'environ 90% en poids d'hydrocarbures à chaînes droites saturées et d'environ 10% en poids d'hydrocarbures monosaturés provenant de cire d'abeilles où la longueur de chaîne desdits hydrocarbures est de $C_{21}$ à $C_{33}$ dans un véhicule pharmaceutique approprié pour l'administration topique.

2. Composition pharmaceutique selon la revendication 1, caractérisé en ce que le mélange d'hydrocarbures provient de cire d'abeilles et la combinaison contient, en outre, moins de 5% en poids calculés sur la quantité totale des constituants de cire d'abeilles de constituants polaires de la cire d'abeilles.

3. Composition pharmaceutique selon la revendication 2, caractérisé en ce que la combinaison comprends 0,1 à 10% en poids, calculés sur le poids de la combinaison de la fraction d'hydrocarbures dérivée de la cire d'abeilles.

4. Composition pharmaceutique selon l'une des revendications 2 ou 3, pour soulager l'inflammation associée à l'acne.

5. Utilisation d'un mélange d'hydrocarbures comprenant d'environ 90% en poids d'hydrocarbures à chaînes droites saturées et d'environ 10% en poids d'hydrocarbures monosaturés où la longueur de chaîne desdits hydrocarbures est de $C_{21}$ à $C_{33}$ pour l'obtention d'une composition pharmaceutique appropriée pour l'administration topique en vue d'une utilisation topique pour soulager l'inflammation associée aux troubles de la peau, notamment l'acne.

**Revendications pour l'Etat Contractant: AT**

1. Procédé d'obtention d'une composition pharmaceutique pur soulager l'inflammation associée aux troubles de la peau, caractérisé en ce qu'un mélange d'hydrocarbures comprenant d'environ 90% en poids d'hydrocarbures à chaînes droites saturées et d'environ 10% en poids d'hydrocarbures monosaturés provenant de cire d'abeilles où la longueur de chaîne desdits hydrocarbures est de $C_{21}$ à $C_{33}$ est combiné à un véhicule pharmaceutique approprié pour l'administration topique.

2. Procédé selon la revendication 1, caractérisé en ce que le mélange d'hydrocarbures provient de cire d'abeilles et qu'en outre, on ajoute à la combinaison moins de 5% en poids calculés sur la quantité totale des constituants de cire d'abeilles de constituants polaires de la cire d'abeilles.

3. Procédé selon la revendication 2, caractérisé en ce qu'on ajoute à la combinaison 0,1 à 10% en poids, calculés sur le poids de la combinaison de la fraction d'hydrocarbures dérivée de la cire d'abeilles.

4. Utilisation d'un mélange d'hydrocarbures comprenant d'environ 90% en poids d'hydrocarbures à chaînes droites saturées et d'environ 10% en poids d'hydrocarbures monosaturés où la longueur de chaîne desdits hydrocarbures est de $C_{21}$ à $C_{33}$ pour l'obtention d'une composition pharmaceutique appropriée pour l'administration topique en vue d'une utilisation topique pour soulager l'inflammation associée aux troubles de la peau, notamment l'acné.